# FASCICULE DE BREVET EUROPEEN

(11) **EP 0 419 312 B1**
(45) Date de publication et mention de la délivrance du brevet: **04.08.1993**
(21) Numéro de dépôt: 90402433.8
(22) Date de dépôt: 04.09.1990
(51) Int. Cl.: C07C 51/36, C07C 59/84, C07B 53/00, A61K 31/19

(54) **Procédé de préparation d'acides aryl-2 propioniques optiquement actifs**
Verfahren zur Herstellung von optisch aktiven 2-Arylpropionsäuren
Method for the production of optically active 2-aryl-propionic acids

(30) Priorité: 05.09.1989 FR 8911568
(43) Date de publication de la demande: 27.03.1991
(73) Titulaire: RHONE-POULENC SANTE, 92160 Antony (FR)
(72) Inventeur: Grosselin, Jean-Michel, F-69340 Francheville-le-Haut (FR)
(74) Mandataire: Pilard, Jacques

(56) Documents cités:
- GB-A- 2 021 583
- CHEMICAL ABSTRACTS, vol. 105, no. 3, 21 juillet 1986, page 675, abrégé no. 24599q, Columbus, Ohio, US; F.ALARIO et al.: "Asymmetric hydrogenation in aqueous-organic two-phase solvent systems using rhodium complexes of sulfonated phosphines"
- GAZETTA CHIMICA ITALIANA, vol. 110, no. 2,3, février, mars 1980, pages 123-127, Societa Chimica Italiana, Roma, I; G. COMISSO et al.: "Enantioselective synthesis and absolute configuration of (+)-alpha-(3-benzoylphenyl)propionic acid"
- CHEMICAL ABSTRACTS, vol. 110, no. 9, 27 février 1989, page 669, abrégé no. 75662r, Columbus, Ohio, US; Y. AMRANI et al.: "Chiral sulfonated phosphines. Syntheses and use as ligands in asymmetric hydrogenation using an aqueous-organic two-phase solvent system"
- CHEMICAL ABSTRACTS, vol. 112, no. 11, 12 mars 1990, page 712, abrégé no. 98158m, Columbus, Ohio, US; V. SUNJIC et al.: "Asymmetric hydrogenation of alpha-arylpropenoic acids catalyzed by rhodium(I) complexes of chiral lignads derived from some monosaccharides"

## Description

La présente invention concerne un procédé de préparation d'acides aryl-2 propioniques optiquement actifs de formule générale : 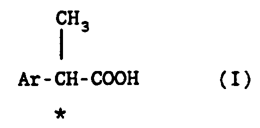
par réduction asymétrique d'un acide aryl-2 acrylique de formule générale :

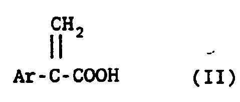

Dans les formules générales (I) et (II), représente un radical aromatique monocyclique ou polycyclique éventuellement substitué ou un radical hétérocyclique aromatique éventuellement substitué.

Plus particulièrement, la présente invention concerne la préparation des énantiomères S des acides aryl-2 propioniques qui présentent des propriétés anti-inflammatoires, analgésiques et/ou antipyrétiques.

Parmi les acides aryl-2 propioniques thérapeutiquement actifs peuvent être cités, par exemple, le kétoprofène, le naproxène, l'ibuprofène, le suprofène, le fénoprofène, le bénoxaprofène, le carprofène, le cicloprofène, le pirprofène, le flurbiprofène et le fluprofène.

Plus particulièrement encore, la présente invention concerne la préparation de l'énantiomère S(+) de l'acide (benzoyl-3 phényl)-2 propionique [ou kétoprofène-S(+)].

Dans le brevet américain US 3 641 127 a été décrite la synthèse du kétoprofène-S(+) par un procédé qui nécessite la réalisation d'un nombre important de phases successives et qui, de ce fait, est d'une application industrielle difficile.

Il est connu, d'après G.Comisso et coll., Gazzetta Chimica Italiana, 110, 123-127 (1980), de réduire énantiosélectivement l'acide (benzoyl-3 phényl)-2 acrylique par l'hydrogène en présence d'un catalyseur à base de rhodium associé à un ligand chiral [(-)-DIOP] en opérant en milieu organique homogène. Cependant la mise en oeuvre de ce procédé ne permet d'obtenir qu'un produit dont la pureté optique n'est pas satisfaisante. Par ailleurs ce procédé ne permet pas un recyclage du catalyseur, ce qui diminue l'intérêt économique du procédé.

Il a maintenant été trouvé, et c'est ce qui fait l'objet de la présente invention, que les acides aryl-2 propioniques optiquement actifs de formule générale (I) peuvent être obtenus avec de bons rendements et avec des excès énantiomériques satisfaisants, par réduction asymétrique en milieu biphasique d'un acide aryl-2 acrylique de formule générale (II).

Selon la présente invention, la réduction asymétrique d'un acide aryl-2 acrylique de formule générale (II) est effectuée par l'hydrogène en présence d'un catalyseur constitué d'un dérivé du sodium associé à un ligand chiral hydrosoluble en opérant dans un milieu hydro-organique bi-phasique.

Les dérivés du rhodium qui conviennent particulièrement bien pour la mise en oeuvre du procédé sont choisis parmi les sels inorganiques ou organiques et les complexes du rhodium comme par exemple RhCl₃, RhBr₃, Rh₂O, Rh₂O₃, Rh(NO₃), Rh(CH₃COO)₃, Rh(CH₃COCHCOCH₃)₃, [RhCl (cyclooctadiène-1,5)]₂. D'un intérêt tout particulier sont [RhCl(cyclooctadiène-1,5)]₂ et RhCl₃.

Les ligands chiraux hydrosolubles sont choisis parmi les phosphines chirales sulfonées qui sont décrites en particulier dans le brevet français FR 83 12468 (2 549 840). De préférence on utilise la (-)-cyclobutanediop-tétrasulfonée de formule : 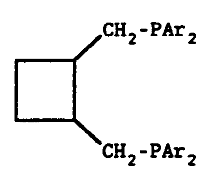
dans laquelle Ar représente le radical de formule :

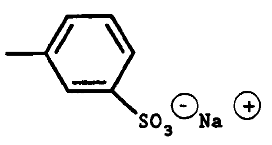

On utilise une quantité de rhodium ou de composé du rhodium telle que le nombre d' atomes-gramme de rhodium élémentaire par litre de solution réactionnelle soit compris entre 10⁻⁴ et 1. De préférence, il est compris entre 0,001 et 0,5.

Pour la bonne mise en oeuvre du procédé, la quantité de phosphine est choisie de telle sorte que le rapport molaire entre le ligand et le composé du rhodium soit compris entre 1 et 100. De préférence le rapport est voisin de 3.

Selon un mode particulier mais non obligatoire de mise en oeuvre du procédé, on peut ajouter une base au mélange réactionnel dans le but d'augmenter la sélectivité. Les bases qui conviennent particulièrement bien sont choisies parmi les hydroxydes, carbonates ou bicarbonates de métaux alcalins ou alcalino-terreux et les amines tertiaires aliphatiques (triéthylamine) ou aromatiques. La quantité de base est choisie de telle sorte que le rapport molaire entre la base et le composé du rhodium soit compris entre 1 et 100.

Généralement, la mise en oeuvre du procédé est effectuée par hydrogénation au moyen d'hydrogène éventuellement sous pression d'un mélange réactionnel obtenu par addition de la solution aqueuse catalytique à une solution de l'acide aryl-2 acrylique de formule générale (II) dans un solvant organique non miscible à l'eau choisi de préférence parmi les esters tel que l'acétate d'éthyle. A la fin de l'hydrogénation, la solution catalytique aqueuse est séparée par décantation, le produit de la réaction étant en phase organique dont il peut être séparé par exemple, par évaporation du solvant.

Généralement, l'hydrogénation est effectuée sous une pression d'hydrogène comprise entre 1 et 100 bars, de préférence voisine de 20 bars, à une température comprise entre -50 et +100 °C, de préférence voisine de 20°C.

La solution aqueuse catalytique qui est séparée en fin de réaction peut être recyclée dans une opération ultérieure d'hydrogénation.

La présente invention concerne également les acides aryl-2 propioniques optiquement actifs, et plus particulièrement le kétoprofène-S(+), lorsqu'ils sont obtenus par la mise en oeuvre du procédé.

Les exemples suivants, donnés à titre non limitatif, illustrent la mise en oeuvre du procédé.

### EXEMPLE 1

Dans un tube de Schlenk, on introduit, sous atmosphère d'argon, 0,060 g de (-)-cyclobutanediop- tétrasulfonée et 5 cm³ d'eau désaérée. On ajoute 1 cm3 d'une solution de [RhCl(cyclooctadiène-1,5)]₂ dans un mélange méthanol-toluène (1-1 en volumes) soit 1,3 x 10⁻⁵ atome-gramme de rhodium. Le rapport molaire entre le ligand et le rhodium est de 3. Après 15 minutes d'agitation à une température voisine de 20°C, la solution aqueuse catalytique jaune est transférée dans une ampoule de verre de 25 cm3 contenant 0,001 mole d'acide (benzoyl-3 phényl)-2 acrylique dissous dans 5 cm3 d'acétate d'éthyle. L'ampoule est placée dans un autoclave de 125 cm3. On établit une pression d' hydrogène de 20 bars. On poursuit l'hydrogénation pendant 17 heures à une température voisine de 20 °C.

A la fin de l'hydrogénation, les phases aqueuse et organique sont séparées par décantation. La phase aqueuse est lavée 3 fois avec 100 cm³ d'acétate d'éthyle. Les phases organiques réunies sont séchées sur sulfate de sodium. Après filtration et évaporation du solvant, on obtient, avec un rendement quantitatif, une huile jaune dont :
- le spectre de résonance magnétique nucléaire du proton montre que le taux de transformation de l'acide (benzoyl-3 phényl)-2 acrylique est de 100 %
- l'analyse par chromatographie liquide chirale à haute performance (HPLC chirale) montre que l'excès énantiomérique en acide (benzoyl-3 phényl)-2 propionique-S(+) est de 71 % (S/R = 85/14).

L'acide (benzoyl-3 phényl)-2 acrylique peut être préparé selon la méthode décrite par G.Comisso et coll., Gazzetta Chimica Italiana, 110, 123-127 (1980).

La (-)-cyclobutanediop-tétrasulfonée peut être préparée selon la méthode décrite dans le brevet français FR 83 12468 (2549840).

### EXEMPLE 2

On opère comme dans l'exemple 1 en opérant en présence d'une quantité de triéthylamine telle que le rapport molaire triéthylamine/composé du rhodium est égal à 5,6.

L'analyse du produit obtenu montre que le taux de transformation de l'acide (benzoyl-3 phényl)-2 acrylique est de 100 % et que l'excès énantiomérique en acide (benzoyl-3 phényl)-2 propionique-S(+) est de 76 % (S/R = 88/12).

La présente invention concerne également les compositions pharmaceutiques anti-inflammatoires, analgésiques et/ou antipyrétiques contenant un acide aryl-2 propionique optiquement actif de formule générale (I), et plus particulièrement le kétoprofène-S(+), obtenu par la mise en oeuvre du procédé, éventuellement sous forme de sel pharmaceutiquement acceptable, éventuellement en association avec un ou plusieurs diluants ou adjuvants pharmaceutiquement acceptables qu'ils soient inertes ou pharmacologiquement actifs.

Les sels pharmaceutiquement acceptables sont généralement choisis parmi les sels avec les bases minérales ou organiques. Comme sels pharmaceutiquement acceptables peuvent être cités les sels de métaux alcalins ou alcalino-terreux (sodium, potassium, calcium), les sels de métaux lourds (cuivre, zinc), les sels d'amines (triéthylamine, triéthanolamine) ou les sels d'amino-acides (lysine, arginine).

Les compositions pharmaceutiques selon l'invention peuvent être des compositions solides, liquides ou semi-liquides qui peuvent être administrées par voie orale, parentérale, rectale ou être utilisées en applications locales ou ophtalmiques.

Comme compositions solides pour administration orale peuvent être cités les comprimés, les poudres ou les granulés qui permettent une libération immédiate ou contrôlée de l' acide aryl-2 propionique optiquement actif de formule générale (II).

Comme compositions liquides pour administration orale peuvent être citées les solutions, les suspensions et les émulsions aqueuses ou non aqueuses.

Comme compositions liquides pour administration parentérale peuvent être citées les solutions stériles aqueuses ou non aqueuses d'un acide aryl-2 propionique optiquement actif de formule générale (I) éventuellement sous forme de sel.

Comme compositions pour administration rectale peuvent être cités les suppositoires ou les gels.

Comme compositions pour application locale peuvent être utilisés les pommages, les onguents, les gels ou les formes transdermales.

Comme composition pour application ophtalmique peuvent être cités les collyres.

En thérapeutique humaine, les doses dépendent de la voie d'administration, de la durée du traitement et de la nature de la maladie à traiter. Généralement les doses journalières sont comprises entre 20 et 500 mg en une ou plusieurs prises.

Les exemples suivants, donnés à titre non limitatif, illustrent des compositions selon l'invention.

### EXEMPLE A

On prépare selon la technique habituelle des comprimés dosés à 25 mg de kétoprofène-S(+) ayant la composition suivante :
- kétoprofène-S(+) 25 mg
- amidon 120 mg
- silice précipitée 27 mg
- stéarate de magnésium 3 mg

### EXEMPLE B

On prépare selon les techniques habituelles une solution injectable contenant :
- kétoprofène-S(+) 25 mg
- arginine 18,3 mg
- acide citrique quantité suffisante pour pH = 6,5
- soluté injectable 2 cm3

### EXEMPLE C

On prépare selon les techniques habituelles un gel contenant :
- kétoprofène-S(+) 0,75 g
- agent gélifiant 0,9 g
- agent de neutralisation 1,8 g
- alcool éthylique 8,1 g
- eau distillée quantité suffisante pour 30 g

## Revendications

1. Procédé de préparation d'un acide aryl-2 propionique optiquement actif de formule générale : 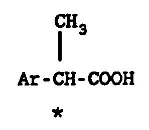 dans laquelle Ar représente un radical aromatique monocyclique ou polycyclique éventuellement substitué ou un radical hétérocyclique aromatique éventuellement substitué par réduction au moyen d'hydrogène d'un acide aryl-2 acrylique de formule générale : 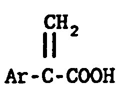 dans laquelle Ar est défini comme précédemment caractérisé en ce que l'on opère en milieu hydro-organique bi-phasique en présence d'un catalyseur constitué d'un dérivé du rhodium associé à un ligand chiral hydrosoluble.

2. Procédé selon la revendication 1 caractérisé en ce que le dérivé du rhodium est choisi parmi les sels inorganiques ou organiques et les complexes du rhodium.

3. Procédé selon la revendication 2 caractérisé en ce que le dérivé du rhodium est choisi parmi [RhCl(cyclooctadiène-1,5)]₂ et RhCl₃.

4. Procédé selon la revendication 1 caractérisé en ce que le ligand chiral hydrosoluble est choisi parmi les phosphines chirales sulfonées.

5. Procédé selon la revendication 4 caractérisé en ce que le ligand chiral hydrosoluble est le (-)-cyclobutanediop-tétrasulfoné.

6. Procédé selon l'une des revendications 1, 2 ou 3 caractérisé en ce que l'on utilise une quantité de rhodium telle que le nombre d'atomes-gramme de rhodium élémentaire par litre de solution réactionnelle est compris entre 10⁻⁴ et 1.

7. Procédé selon l'une des revendications 1, 4 ou 5 caractérisé en ce que le rapport molaire entre le ligand et le composé du rhodium est compris entre 1 et 100.

8. Procédé selon l'une des revendications 1 à 7 caractérisé en ce que l'on opère sous une pression d'hydrogène comprise entre 1 et 100 bars.

9. Procédé selon l'une des revendications 1 à 8 caractérisé en ce que l'on opère à une température comprise entre -50 et +100°C.

10. Procédé selon l'une des revendications 1 à 9 caractérisé en ce que l'on opère en outre en présence d'une base minérale ou organique.

11. Procédé selon la revendication 10 caractérisé en ce que la base est la triéthylamine.

12. Procédé selon l'une des revendications 1 à 11 pour la préparation de l'acide (benzoyl-3 phényl)-2 propionique-S(+) par réduction de l'acide (benzoyl-3 phényl)-2 acrylique.

## Claims

1. Process for preparing an optically active 2-arylpropionic acid of general formula: 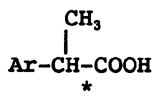 in which Ar represents an optionally substituted monocyclic or polycyclic aromatic radical or an optionally substituted aromatic heterocyclic radical, by the reduction by means of hydrogen of a 2-arylacrylic acid of general formula: 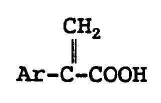 in which Ar is defined as above, characterised in that the reaction is performed in a two-phase aqueous-organic medium in the presence of a catalyst consisting of a rhodium derivative combined with a water-soluble chiral ligand.

2. Process according to claim 1, characterised in that the rhodium derivative is selected from the inorganic or organic salts and the complexes of rhodium.

3. Process according to claim 2, characterised in that the rhodium derivative is selected from [RhCl(1,5-cyclooctadiene)]₂ and RhCl₃.

4. Process according to claim 1, characterised in that the water-soluble chiral ligand is selected from sulphonated chiral phosphines.

5. Process according to claim 4, characterised in that the water-soluble chiral ligand is tetrasulphonated (-)-cyclobutaneDIOP.

6. Process according to one of claims 1, 2 or 3, characterised in that a quantity of rhodium is used, such that the number of gram-atoms of elemental rhodium per litre of reaction solution is between 10⁻⁴ and 1.

7. Process according to one of claims 1, 4 or 5, characterised in that the mole ratio of the ligand to the rhodium compound is between 1 and 100.

8. Process according to one of claims 1 to 7, characterised in that the reaction is performed under a hydrogen pressure of between 1 and 100 bars.

9. Process according to one of claims 1 to 8, characterised in that the reaction is performed at a temperature of between -50 and +100 °C.

10. Process according to one of claims 1 to 9, characterised in that the reaction is performed, in addition, in the presence of an inorganic or organic base.

11. Process according to claim 10, characterised in that the base is triethylamine.

12. Process according to one of claims 1 to 11, for the preparation of (S)-(+)-2-(3-benzoylphenyl)-propionic acid by the reduction of 2-(3-benzoylphenyl)-acrylic acid.

## Patentansprüche

1. Verfahren zur Herstellung einer optisch aktiven 2-Arylpropionsäure der allgemeinen Formel 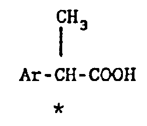 worin Ar für einen gegebenenfalls substituierten monocyclischen oder polycyclischen aromatischen Rest oder einen gegebenenfalls substituierten aromatischen heterocyclischen Rest steht, durch Reduktion mit Hilfe von Wasserstoff einer 2-Arylacrylsäure der allgemeinen Formel 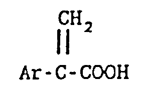 worin Ar wie vorstehend definiert ist, dadurch gekennzeichnet daß man in einem zweiphasigen wäßrig-organischen Milieu in Gegenwart eines Katalysators arbeitet, der aus einem Rhodiumderivat besteht, welches mit einem wasserlöslichen chiralen Liganden assoziiert ist.

2. Verfahren gemäß Anspruch 1, dadurch gekennzeichnet, daß das Rhodiumderivat unter den anorganischen oder organischen Salzen und den Komplexen des Rhodiums ausgewählt wird.

3. Verfahren gemäß Anspruch 2, dadurch gekennzeichnet, daß das Rhodiumderivat unter [RhCl(cyclo-1,5-octadien)]₂ und RhCl₃ ausgewählt wird.

4. Verfahren gemäß Anspruch 1, dadurch gekennzeichnet, daß der wasserlösliche chirale Ligand unter sulfonierten chiralen Phosphinen ausgewählt wird.

5. Verfahren gemäß Anspruch 4, dadurch gekennzeichnet, daß der wasserlösliche chirale Ligand (-)-Cyclobutandiop-tetrasulfoniert ist.

6. Verfahren gemäß einem der Ansprüche 1, 2 oder 3, dadurch gekennzeichnet, daß man eine derartige Rhodiummenge verwendet, daß die Anzahl Grammatome elementares Rhodium je Liter Reaktionslösung zwischen 10⁻⁴ und 1 beträgt.

7. Verfahren gemäß einem der Ansprüche 1, 4 oder 5, dadurch gekennzeichnet, daß das Molverhältnis zwischen dem Liganden und der Rhodiumverbindung zwischen 1 und 100 liegt.

8. Verfahren gemäß einem der Ansprüche 1 bis 7, dadurch gekennzeichnet, daß man unter einem Wasserstoffdruck zwischen 1 und 100 bar arbeitet.

9. Verfahren gemäß einem der Ansprüche 1 bis 8, dadurch gekennzeichnet, daß man bei einer Temperatur zwischen -50 und +100°C arbeitet.

10. Verfahren gemäß einem der Ansprüche 1 bis 9, dadurch gekennzeichnet, daß man außerdem in Anwesenheit einer mineralischen oder organischen Base arbeitet.

11. Verfahren gemäß Anspruch 10, dadurch gekennzeichnet, daß die Base Triethylamin ist.

12. Verfahren gemäß einem der Ansprüche 1 bis 11 zur Herstellung der S(+)-2-(3-Benzoylphenyl)-propionsäure durch Reduktion der 2-(3-Benzoylphenyl)-acrylsäure.
